(19) Europäisches Patentamt
European Patent Office
Office européen des brevets

(11) Veröffentlichungsnummer: **0 068 282**
**B2**

(12) # NEUE EUROPÄISCHE PATENTSCHRIFT

(45) Veröffentlichungstag der neuen Patentschrift: **08.03.89**

(51) Int. Cl.⁴: **C 07 C 29/62**, C 07 C 31/36, C 07 C 31/42

(21) Anmeldenummer: **82105226.3**

(22) Anmeldetag: **15.06.82**

(54) **Verfahren zur Herstellung von Bromhydrinen aus Polyolen.**

(30) Priorität: **27.06.81 DE 3125338**

(43) Veröffentlichungstag der Anmeldung: **05.01.83 Patentblatt 83/1**

(45) Bekanntmachung des Hinweises auf die Patenterteilung: **13.02.85 Patentblatt 85/7**

(45) Bekanntmachung des Hinweises auf die Entscheidung über den Einspruch: **08.03.89 Patentblatt 89/10**

(84) Benannte Vertragsstaaten: **BE DE FR GB IT**

(56) Entgegenhaltungen:
**DE-A-2 741 407**
**FR-A-2 230 604**
**FR-A-2 241 535**
**US-A-2 144 612**
**US-A-3 932 541**
**US-A-4 154 966**

(73) Patentinhaber: **BAYER AG, Konzernverwaltung RP Patentabteilung, D-5090 Leverkusen 1 Bayerwerk (DE)**

(72) Erfinder: **König, Klaus, Dr., Heymannstrasse 50, D-5090 Leverkusen (DE)**
Erfinder: **Schmidt, Manfred, Dr., Zeisigstrasse 5, D-4047 Dormagen (DE)**



LIBER, STOCKHOLM 1989

**Beschreibung**

Es sind bereits verschiedene Verfahren zur Einführung von Brom in Polyole bekanntgeworden. Verfahren, bei denen Phosphorbromide oder Thionylbromid als Bromierungsagens eingesetzt werden oder die über Benzolsulfonsäureester als Zwischenstufe verlaufen sind in ihrer Durchführung sehr aufwendig, erfordern teure Reagentien und werden deshalb technisch nicht angewendet.

Andere Verfahren, bei denen wasserfreier Bromwasserstoff und organische Lösungsmittel, wie Toluol oder Perchlorethylen, eingesetzt werden, benötigen hohe Überschüsse an Bromwasserstoff und/oder die Anwendung höherer Temperaturen und/oder Drücke, um einigermaßen akzeptable Ausbeuten zu erzielen. Diese Verfahren haben ebenfalls keine technische Bedeutung.

Auch Gemische aus Bromwasserstoff und Eisessig wurden schon eingesetzt. Dabei erwies es sich als notwendig, das Reaktionswasser durch Zugabe von Essigsäureanhydrid oder Acetylbromid zu binden. Der Nachteil hierbei ist, daß nach der Reaktion alle nicht durch Brom substituierten Hydroxylgruppen in Form von Essigsäureestern vorliegen, die erst in einer Umesterungsreaktion wieder freigesetzt werden müssen.

Verfahren, in denen wäßriger Bromwasserstoff eingesetzt wird, erfordern, um gute Ergebnisse und annehmbare Reaktionszeiten realisieren zu können, wäßrige Bromwasserstofflösungen mit Konzentrationen an HBr oberhalb der handelsüblichen 48 %-igen Bromwasserstoffsäure. Das bedeutet, für derartige Verfahren muß zunächst die handelsübliche Bromwasserstoffsäure durch Einleiten von gasformigem HBr aufkonzentriert werden. Trotzdem sind noch hohe Reaktionstemperaturen und/oder das Arbeiten unter Druck in Autoklaven erforderlich.

Als derzeitig gunstigste Verfahren können diejenigen angesehen werden, bei denen die Polyole in Gegenwart größerer Mengen Eisessig mit 48- bis 66 %-iger wäßriger Bromwasserstofflösung bei Normaldruck umgesetzt werden (s. z. B. J. Org. Chem., 30, 1945 und 3308 [1965]). Nachteilig ist hierbei jedoch, daß auch hier die erhaltenen Bromhydrine ganz oder teilweise an den noch vorhandenen Hydroxylgruppen acetyliert sind und die freien Bromhydrine erst in einer nachgeschalteten Umesterungsreaktion erhalten werden können. Hierzu setzt man einen kurzkettigen Alkohol, z. B. Methanol, und einen sauren Katalysator zu und destilliert den gebildeten Essigsäureester und überschüssigen kurzkettigen Alkohol ab. Anschließend muß das so erhaltene rohe Bromhydrin noch durch Umkristallisation gereinigt werden.

Die Aufwendigkeit dieser Arbeitsweise geht aus der DE-OS-2 440 612 hervor. Gemäß Beispiel 1 dieser DE-OS werden 40 Mol HBr in Form einer wäßrigen, 62 %-igen Losung mit 20 Mol Pentaerythrit in Gegenwart von 10 Mol Essigsäure bei etwa 120°C umgesetzt. Während der Reaktion destilliert das mit der HBr-Lösung eingebrachte und in der Bromierungsreaktion gebildete Wasser zusammen mit etwas Essigsäure ab. Der Rest der Essigsäure ist in Form von Acetaten an das bromierte Pentaerythrit gebunden. Um das bromierte Pentaerythrit von diesen Acetatgruppen zu befreien, setzt man nach dem Abkühlen 2 Methanol zu und eine katalytische Menge 62 %-iger HBr-Lösung, worauf man zum Sieden erhitzt. Man destilliert ein Gemisch aus Methanol und Essigsäureniethylester ab. Bevor das überschüssige Methanol vollständig entfernt ist, wird mit methanolischer Ammoniaklösung neutralisiert. Anschließend wird Aktivkohle zugesetzt, erneut erhitzt und heiß über ein Filterhilfsmittel filtriert. Danach werden letzte Methanolreste durch Eindampfen zur Trockene entfernt. Das so erhaltene Rohprodukt wird aus Trichlorethylen umkristallisiert. Zusammengefaßt ist also festzustellen, daß die Nachbehandlung des Reaktionsproduktes mehr Aufwand erfordert als die eigentliche Bromierung.

Aus US-A-4 154 966 ist ein Verfahren bekannt geworden, bei dem Dibromnespentylglykol durch Umsetzung von Pentaerythriol mit Bromwasserstoff in einem inerten Lösungsmittel umgesetzt wird. Das Verfahren hat den Nachteil daß mit inerten organischen Lösungsmitteln gearbeitet werden muß und daß eine Neutralisation erforderlich ist.

Es wurde nun ein Verfahren zur Herstellung von Bromhydrinen durch Umsetzung eines Polyols der Formel

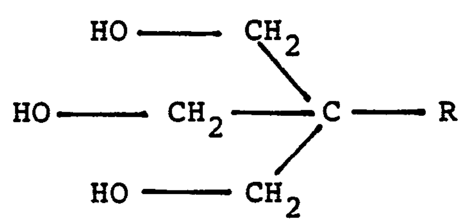

$$R = CH_2OH,\ CH_3 \text{ oder } C_2H_5$$ ,

mit HBr in Gegenwart einer katalytischen Menge einer niedermolekularen organischen Säure und unter destillativer Entfernung von Wasser während der Reaktion gefunden, das dadurch gekennzeichnet ist, daß man eine wäßrige HBr-Lösung einsetzt und das Verfahren in Abwesenheit eines inerten organischen Lösungsmittels und ohne anschließende Neutralisation durchgeführt wird.

Zum Einsatz in das erfindungsgemäße Verfahren sind die Polyole Pentaerythrit, Trimethylolethan und Trimethylolpropan geeignet. In diesen können, je nach der Menge der eingesetzten HBr-Lösung, eine oder

mehrere OH-Gruppen durch Brom ersetzt und somit die entsprechenden Mono- oder Polybromhydride oder Gemische davon erhalten werden. Die einzusetzenden Polyole können auf beliebige Weise hergestellt worden sein und in handelsublicher Reinheit eingesetzt werden.

Die Konzentration der wäßrigen HBr-Lösung kann in weiten Grenzen schwanken. Aus wirtschaftlichen Gründen ist der Einsatz von gut zugänglichen wäßrigen HBr-Lösungen bevorzugt, beispielsweise von solchen mit HBr-Gehalten im Bereich von 20 bis 50 Gew.-%. Es können aber auch höher und niederer konzentrierte HBr-Lösungen eingesetzt werden. Vorzugsweise setzt man 40 bis 50 gew.-%-ige wäßrige HBr-Lösung ein, besonders bevorzugt die handelsübliche, etwa 48 gew.-%-ige wäßrige HBr-Lösung. Je nach der Konzentration der eingesetzten HBr-Lösung können die erforderlichen Reaktionszeiten variieren.

Die Menge HBr-Lösung richtet sich im wesentlichen nach der Anzahl umzusetzender OH-Gruppen. Beispielsweise kann man pro Mol umzusetzender OH-Gruppen 0,9 bis 2,0 Mol HBr einsetzen. Vorzugsweise setzt man 10 bis 1,25 Mol HBr pro Mol umzusetzender OH-Gruppen ein.

Geeignete Reaktionstemperaturen sind beispielsweise solche im Bereich 20 bis 160°C. Vorzugsweise arbeitet man bei 80 bis 150°C. Man kann auch so vorgehen, daß man die Reaktion bei relativ niedrigen Temperaturen beginnt, z. B. bei Raumtemperatur, und dann auf höhere Temperaturen, beispielsweise 80 bis 150°C übergeht.

Der Druck wird so gewählt, daß während der Reaktion gebildetes und sonstiges vorhandenes, insbesondere mit der HBr-Lösung eingebrachtes Wasser, abdestilliert. Dabei kann auch die eingesetzte organische Säure und gegebenenfalls vorhandenes überschüssiges HBr ganz oder teilweise, insbesondere gegen Ende der Reaktion, mit übergehen. Vorzugsweise erfolgt dieses Abdestillieren über eine Kolonne. Je nach der angewendeten Reaktionstemperatur können die Drücke im Bereich von beispielsweise 0,2 bis 2 bar, vorzugsweise im Bereich 0,3 bis 1,5 bar, liegen. Besonders bevorzugt arbeitet man bei Normaldruck und destilliert das Wasser bei ca. 100°C über, wobei dann Sumpftemperaturen im Bereich von etwa 110 bis 130°C auftreten.

Es ist ein wesentliches Merkmal des erfindungsgemäßen Verfahrens, daß es im Gegensatz zu bisher bekannten Verfahren nicht in einem Medium aus Essigsäure, sondern nur unter Einsatz einer katalytischen Menge einer niedermolekularen organischen Säure durchgeführt wird. Als niedermolekulare organische Säuren kommen beispielsweise Carbonsäuren mit 1 bis 4 C-Atomen in Frage, bei denen die Alkyl-Wasserstoff-Atome auch teilweise oder ganz durch Halogenatome ersetzt sein können. Als Beispiele seien genannt: Ameisensäure, Essigsäure, Propionsäure, n-Buttersäure, i-Buttersäure, Monochloressigsäure, Dichloressigsäure, Trichloressigsäure, Monochlorpropionsäure, Dichlorpropionsäure, Monofluoressigsäure, Difluoressigsäure und Trifluoressigsäure. Bevorzugt wird Essigsäure eingesetzt.

Die Menge, in der eine niedermolekulare organische Säure eingesetzt wird, kann beispielsweise 0,1 bis 3 Gew.-%, bezogen auf das Gesamtgewicht des Reaktionsansatzes, betragen. Vorzugsweise beträgt diese Menge 0,1 bis 2 Gew.-%.

Die Reaktionszeit für die erfindungsgemäße Umsetzung liegt im allgemeinen zwischen 5 und 20 Stunden. Die Reaktionszeit hängt insbesondere von der Menge eingesetzter niedermolekularer organischer Säure, von der Art der niedermolekularen organischen Säure, von der Konzentration der wäßrigen HBr-Lösung und von der Geschwindigkeit ab, mit der das Wasser abdestilliert wird.

Das erfindungsgemäße Verfahren kann diskontinuierlich und kontinuierlich durchgeführt werden. Für die kontinuierliche Durchführung ist beispielsweise eine Reaktionskaskade geeignet.

Bei einer bevorzugten Ausführungsform des erfindungsgemäßen Verfahrens wird so gearbeitet, daß man Pentaerythrit, Trimethylolethan oder Trimethylolpropan nach Zusatz von 0,1 bis 2 Gew.-% Essigsäure, bezogen auf das Gesamtgewicht des Reaktionsansatzes mit handelsüblicher wäßriger, ca. 48 %-iger HBr-Lösung in einem Reaktionsbehälter umsetzt, der mit einer Füllkörperkolonne mit Rücklaufteiler versehen ist. Pro Mol umzusetzender OH-Gruppen setzt man 1,0 bis 1,25 Mol HBr ein. Man erhitzt den Inhalt des Reaktionsbehälters bei Normaldruck auf etwa 120 bis 130°C und regelt die Wärmezufuhr und das Rücklaufverhältnis so, daß am Kolonnenkopf zunächst bei 100°C nur Wasser abgenommen werden kann. Gegen Ende der Reaktion läßt man die Abnahmetemperatur auf ca. 120°C ansteigen. Man kann selbstverständlich auch unter vermindertem Druck und entsprechend erniedrigter Destillationstemperatur arbeiten. So gehen außer Wasser bis zu 90 % der eingesetzten Essigsäure und Reste von nichtumgesetztem HBr über.

Nach dem erfindungsgemäßen Verfahren erhält man Bromhydrine in Ausbeuten von etwa 85 bis 90 % der Theorie. Das erhaltene Produkt enthält im allgemeinen geringe Mengen an Nebenprodukten, hauptsächlich die um ein Bromatom reicheren und ärmeren Bromhydrine. Es kann häufig direkt so wie es erhalten wird verwendet werden. Wenn reinere Produkte gewünscht werden, so kann eine Reinigung beispielsweise durch Destillation im Vakuum vorgenommen werden, bei der es allerdings im allgemeinen nicht gelingt, die Nebenprodukte vollständig zu entfernen. Hochreine Bromhydrine (über 99 % rein) können durch Umkristallisation, beispielsweise aus Trichlorethylen oder Chloroform erhalten werden.

Das erfindungsgemäße Verfahren bietet gegenüber den Verfahren des Standes der Technik insbesondere folgende Vorteile: Die Bromhydrine fallen so rein an, daß sie nach der destillativen Entfernung aus dem Reaktionsgemisch nicht weiter gereinigt werden müssen. Die bisher notwendige, der eigentlichen Umsetzung nachgeschaltete Umesterungsreaktion mit einem Alkohol entfällt, damit wird eine Reaktionsstufe eingespart. Es wird nur noch eine sehr kleine Menge organischer Säure benötigt.

Es ist als ausgesprochen überraschend anzusehen, daß es erfindungsgemäß gelingt, mit nur sehr geringen Mengen an organischen Säuren so gute Ergebnisse zu erhalten, da bisher wesentlich größere Mengen an

Essigsäure für erforderlich erachtet wurden.

Die erfindungsgemäß herstellbaren Bromhydrine sind bekannte Produkte, die bekannterweise für die Flammfestausrüstung von Kunststoffen Anwendung finden können (s. z B. EP-OS-0 018 176).

Die erfindungsgemäß hergestellten Bromhydrine lassen sich darüber hinaus leicht modifizieren. So finden sie beispielsweise in Form ihrer Carboxylierungsprodukte, Carbonsäureester, Phosphorsäureester und Carbonate vielseitige Anwendung als Flammschutzmittel (s. beispielsweise DE-OS-2 157 214 und DE-OS-2 701 856).

Die folgenden Beispiele erläutern das erfindungsgemäße Verfahren.

**Beispiele**

**Beispiel 1**

Herstellung von 2,2-Bis-(bromomethyl)-propan-1,3-diol

In einem 10-l-Rundkolben, der mit Heizpilz, Rührer und einer 50 cm langen Füllkörperkolonne mit einem Rücklaufteiler versehen war, wurden 2720 g (20 Mol) Pentaerythrit, 6800 g 48 %-ige wäßrige HBr-Lösung (40,3 Mol) und 150 g Essigsäure vorgelegt. Nachdem die Temperatur am Kolonnenkopf bei Normaldruck 100 bis 102°C erreicht hatte, wurde bei dieser Temperatur das Wasser abdestilliert. Die Sumpftemperatur betrug hierbei 125 bis 130°C. Nach 6 Stunden war die Hauptmenge des Wassers abgenommen. Während die Sumpftemperatur langsam auf 150°C anstieg wurde das Rücklaufverhältnis auf 2 : 1 eingeregelt (2 Teile Abnahme, 1 Teil Rücklauf). Es wurden so in den nächsten 3 Stunden die letzten Reste Wasser abdestilliert. Leichtflüchtige Nebenprodukte wurden dann im Wasserstrahlvakuum bei 10 mbar zusammen mit den letzten Resten Wasser abdestilliert. Es wurden so 4460 g Destillat erhalten, das 100 % der berechneten Menge Wasser, 79 % der eingesetzten Essigsäure und 1,5 % des eingesetzten HBr enthielt. Außerdem waren in dem Destillat noch ca. 35 g nicht näher identifizierte flüchtige organische Verbindungen enthalten.

Der Rückstand wurde im Vakuum bei 120 bis 130°C und 0,2 mbar destilliert. Es wurden so 4575 g, das entspricht 87 % der Theorie, eines wasserklaren Produktes erhalten, welches alsbald kristallisierte. Das so erhaltene Produkt hatte folgende Zusammensetzung (Bestimmung nach HPLG = Hochdruckflüssigkeitschromatographie):

2,4 Gew.-% 2-Bromomethyl-2-hydroxymethyl-propan-1,3-diol
84,2 Gew.-% 2,2-Bis-(bromomethyl)-propan-1,3-diol
12,1 Gew.-% 2,2,2-Tris-(bromomethyl)-ethanol

Das so erhaltene Produkt konnte ohne weitere Reinigung verwendet werden.

**Beispiel 2**

Vergleichsbeispiel entsprechend DE-OS-2 440 612, jedoch mit 48 %-iger wäßriger HBr-Lösung

20 Mol Bromwasserstoff in Form einer 48 %-igen wäßrigen Lösung wurden mit 10 Mol Pentaerythrit und 5 Mol Eisessig gemischt und in einer dem Beispiel 1 analogen Apparatur zum Rückfluß erhitzt. Innerhalb von 7 Stunden wurde bei einer Sumpftemperatur von 120 bis 150°C und einer Kopftemperatur von 101 bis 102°C das eingesetzte und entstandene Wasser bei Normaldruck abgenommen. Um das in Form des Acetats vorliegende bromierte Pentaerythrit freizusetzen, wurde eine Umesterung mit Methanol durchgeführt. Hierzu wurden nach dem Abkühlen 1 l Methanol und 2 ml 48 %-ige HBr-Lösung zugefügt, und der Ansatz zum Sieden am Rückfluß gebracht. Dann wurde eine Mischung aus Methanol und Essigsäuremethylester abdestilliert. Nach 2 Stunden hatte die Temperatur am Kolonnenkopf den Siedepunkt des Methanols erreicht. Danach wurde abgekühlt, mit 4 n methanolischer Ammoniaklösung neutralisiert und 80 g Aktivkohle zugesetzt. Dann wurde weitere 45 Minuten unter Rückfluß gekocht und danach in der Hitze über ein Filterhilfsmittel (Mikrofil) filtriert. Das schwachgelbgefärbte Filtrat wurde anschließend zur Trockne eingedampft. So wurden 2160 g (82 %) eines rohen Produktes mit folgender Zusammensetzung erhalten (bestimmt durch HPLG):

1 Gew.-% 2-Bromomethyl-2-hydroxymethyl-propan-1,3-diol
78 Gew.-% 2,2-Bis-(bromomethyl)-propan-1,3-diol
20 Gew.-% 2,2,2-Tris-(bromomethyl)-ethanol

**Beispiel 3**

Herstellung von 2,2-Bis-(bromomethyl)-propan-1,3-diol mit Propionsäure als Katalysator

In entsprechender Weise wie in Beispiel 1 beschrieben wurden 10 Mol Pentaerythrit und 20 Mol HBr in Form einer wäßrigen 48 %-igen Lösung unter Einsatz von 2 Gew.-% Propionsäure umgesetzt. Bei einer Kopftemperatur von 100 bis 102°C und einer Sumpftemperatur von 120 bis 140°C wurde innerhalb von 7 Stunden das gesamte eingebrachte und gebildete Wasser abdestilliert. Nach Destillation des Rückstandes im Vakuum wurden 2332 g (89 % der Theorie) eines Produktes mit folgender Zusammensetzung erhalten (bestimmt durch HPLG):

1,7 Gew.-% 2-Bromomethyl-2-hydroxymethyl-propan-1,3-diol
83,5 Gew.-% 2,2-Bis-(bromomethyl)-propan-1,3-diol
13,8 Gew.-% 2,2,2-Tris-(bromomethyl)-ethanol

**Beispiel 4**

Herstellung von 2,2-Bis-(bromomethyl)-propan-1,3-diol unter Verwendung von 24 gew.-%-iger HBr-Lösung

680 g Pentaerythrit, 3400 g 24 %-ige wäßrige Bromwasserstoffsäure und 40 g Essigsäure wurden in der Weise umgesetzt wie in Beispiel 1 beschrieben. Nach 5 Stunden Reaktionszeit waren 2350 g Wasser überdestilliert. Nach Zugabe von weiteren 12 g Essigsäure wurde das restliche Wasser innerhalb von 2 Stunden abdestilliert. Nach Destillation des Rückstandes im Vakuum wurden 82 % eines Produktes folgender Zusammensetzung erhalten (bestimmt durch HPLG):

7,9 Gew.-% 2-Bromomethyl-2-hydroxymethyl-propan-1,3-diol
84,2 Gew.-% 2,2-Bis-(bromomethyl)-propan-1,3-diol
5,6 Gew.-% 2,2,2-Tris-(bromomethyl)-ethanol

**Beispiel 5**

Herstellung von 2-Bromomethyl-2-hydroxymethyl-butanol-1

In einem 1-l-Dreihalskolben wurden 268 g (2 Mol) Trimethylolpropan in 374 g (entsprechend 2,2 Mol) 48 %-iger wäßriger Bromwasserstoffsäure und 6 g Essigsäure gelöst und etwa 3 Stunden bei Raumtemperatur gerührt. Danach wurde bei 400 mbar zum Sieden erhitzt und über eine Füllkörperkolonne mit Rücklaufteiler im Verlauf von 5 Stunden bis zu einer Sumpftemperatur von 130°C Wasser abdestillert. Die restliche Bromwasserstoffsäure wurde bei 20 mbar abgezogen. Es blieben 394 g Rückstand, der nach gaschromatographischer Analyse zu 80 % aus 2-Bromomethyl-2-hydroxymethyl-butanol-1 bestand. Durch Umkristallisation aus Toluol und Wasser wurden 270 g (68 % der Theorie) der reinen Verbindung erhalten.

**Beispiel 6**

Herstellung von 2,2-Bis-(bromomethyl)-butanol-1

In einem 10-l-Dreihalskolben wurden 2680 g (20 Mol) Trimethylolpropan, 8500 g (50 Mol) 48 %-ige wäßrige Bromwasserstoffsäure und 300 g Essigsäure vermischt und über Nacht bei Raumtemperatur gerührt. Dann wurden bei 400 mbar über eine Füllkörperkolonne innerhalb von 8 Stunden bei einem Rücklaufverhältnis von 2 : 1 3 l Destillat abgenommen. Nachdem die Hauptmenge Bromwasserstoffsäure reagiert hatte, wurde bei einem Druck von 1 bar bis zu einer Sumpftemperatur von 150°C weiter Wasser abdestilliert. Mitdestillierte Produktanteile, die sich in der Vorlage abschieden, wurden vor der anschließenden Destillation mit der Sumpf vereinigt. Danach wurde der Rückstand im Vakuum bei 135 bis 140°C und 16 mbar destilliert. Es wurden 4559 g eines langsam kristallisierenden Öles erhalten, welches nach gaschromatographischer Analyse zu 90 % aus 2,2-Bis-(bromomethyl)-butanol-1 bestand.

**Beispiel 7**

Herstellung von 2 Bromomethyl-2-hydroxymethyl-propan-1,3-diol

In einer dem Beispiel 1 analogen Apparatur von 2 Inhalt wurden 480 g (4 Mol) Trimethylolethan, 748 g (4.4 Mol) 48 %-ige wäßrige HBr-Lösung und 12 g Essigsäure (1 %) vorgelegt. Diese Mischung wurde 3 Stunden bei Raumtemperatur gerührt und anschließend bei 300 mbar bis zu einer Sumpftemperatur von 130°C Wasser abgenommen. Die Kolonnenkopftemperatur betrug hierbei etwa 80°C. Nach 9 Stunden war das gesamte Wasser übergegangen. Das verbleibende Produkt wurde bei 0,7 mbar und einem Siedepunkt von 120 bis 130°C destilliert. Es wurden 660 g (90 %) 2-Bromomethyl-2-methyl-propan-1,3-diol erhalten, welches 10 % der entsprechenden dibromierten Verbindung und ca. 4 % Ausgangsprodukt enthielt.

**Beispiel 8**

Herstellung von 2,2,2-Tris-(bromomethyl)-ethanol-1

In einer Beispiel 1 analogen Apparatur wurden 10 Mol (1360 g) Pentaerythrit, 37,5 Mol HBr in Form der wäßrigen 48 %-igen Lösung und 100 g Essigsäure vorgelegt. Nachdem 3 Stunden unter Rückfluß zum Sieden erhitzt wurde, konnte bei einer Kopftemperatur von 100 bis 102°C entstandenes und eingebrachtes Wasser abdestilliert werden. Innerhalb der nächsten 9 Stunden destillierte die gesamte Menge Wasser und der überschüssige Bromwasserstoff ab. Man erhielt so 3985 g Destillat. Die Sumpftemperatur stieg in diesem Zeitraum von 120 auf 145°C an. Nach Destillation im Vakuum bei 110 bis 120°C und 0,5 mbar fielen 2959 g (91 %) 2,2,2-Tris-(bromomethyl)-ethanol-1 folgende Zusammensetzung an (nach HPLG):

1,5 Gew.-% 2-Bromomethyl-2-hydroxymethyl-propan-1,3-diol
9,3 Gew.-% 2,2-Bis-(bromomethyl)-propanl-1,3-diol
88 Gew.-% 2,2,2-Tris-(bromomethyl)-ethanol

**Patentansprüche**

1. Verfahren zur Herstellung von Bromhydrinen durch Umsetzung eines Polyols der Formel

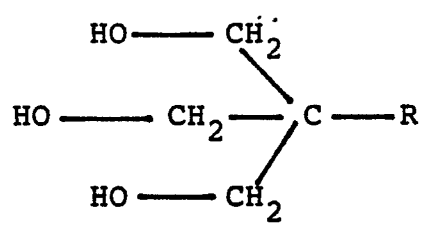

$$R = CH_2OH,\ CH_3 \text{ oder } C_2H_5$$

mit HBr in Gegenwart einer katalytischen Menge einer niedermolekularen organischen Säure und unter destillativer Entfernung von Wasser während der Reaktion, dadurch gekennzeichnet, daß man eine wäßrige HBr-Lösung einsetzt und daß das Verfahren in Abwesenheit eines inerten organischen Lösungsmittels und ohne anschließende Neutralisation durchgeführt wird.

2. Verfahren nach Anspruch 1, dadurch gekennzeichnet, daß man die niedermolekulare organische Säure in einer Menge von 0,1 bis 3 Gew.-%, bezogen auf das Gesamtgewicht des Reaktionsansatzes, einsetzt.

3. Verfahren nach Ansprüchen 1 und 2, dadurch gekennzeichnet, daß man eine Carbonsäure mit 1 bis 4 C-Atomen einsetzt.

4. Verfahren nach Ansprüchen 1 bis 3, dadurch gekennzeichnet, daß man Essigsäure einsetzt.

5. Verfahren nach Ansprüchen 1 bis 4, dadurch gekennzeichnet, daß man eine 20 bis 50 gew.-%-ige wäßrige HBr-Lösung einsetzt.

6. Verfahren nach Ansprüchen 1 bis 5, dadurch gekennzeichnet, daß pro Mol umzusetzender OH-Gruppen 0,9 bis 2,0 Mol HBr einsetzt.

7. Verfahren nach Ansprüchen 1 bis 6, dadurch gekennzeichnet, daß man die Umsetzung bei Temperaturen im Bereich 20 bis 160°C durchgeführt.

## Claims

1. Process for the preparation of bromohydrins by reaction of a polyol of the formula

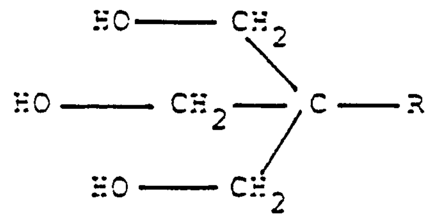

R = $CH_2OH$, $CH_3$ or $C_2H_5$

with HBr in the presence of a catalytic amount of a low molecular weight organic acid and with the removal of water by distillation during the reaction, characterised in that an aqueous HBr solution is used and in that the process is carried out in the absence of an inert organic solvent and without subsequent neutralisation.

2. Process according to Claim 1, characterised in that the low molecular weight organic acid is employed in an amount of 0.1 to 3 % by weight relative to the total weight of the reaction batch.

3. Process according to Claims 1 and 2, characterised in that a carboxylic acid with 1 to 4 C atoms is employed.

4. Process according to Claims 1 to 3, characterised in that acetic acid is employed.

5. Process according to Claims 1 to 4, characterised in that a 20 to 50 % by weight aqueous HBr solution is employed.

6. Process according to Claims 1 to 5, characterised in that 0.9 to 2.0 mols of HBr is employed per mol of OH grups to be reacted.

7. Process according to Claims 1 to 6, characterised in that the reaction is carried out at temperatures in the range from 20 to 160° C.

## Revendications

1. Procédé de production de bromhydrines par réaction d'un polyol de formule

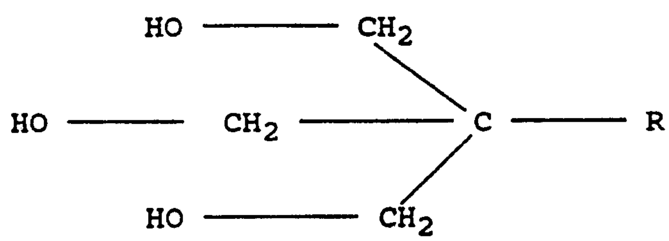

avec HBr en présence d'une quantité catalytique d'un acide organique de bas poids moléculaire et avec élimination par distillation d'eau pendant la réaction, caractérisé en ce qu'on utilise une solution aqueuse de HBr et en ce qu'on le met en oeuvre en l'absence d'un solvant organique inerte et sans neutralisation ultérieure.

2. Procédé suivant la revendication 1, caractérisé en ce qu'on utilise l'acide organique de bas poids moléculaire en une quantité de 0,1 à 3 % en poids par rapport au poids total du mélange réactionnel.

3. Procédé suivant les revendications 1 et 2, caractérisé en ce qu'on utilise un acide carborylique ayant 1 à 4 atomes de carbone.

4. Procédé suivant les revendications 1 à 3, caractérisé en ce qu'on utilise l'acide acétique.

5. Procédé suivant les revendications 1 à 4, caractérisé en ce qu'on utilise une solution aqueuse de HBr à 20-50 % en poids.

6. Procédé suivant les revendications 1 à 5, caractérisé en ce qu'on utilise 0,9 à 2,0 moles de HBr par mole de groupes OH devant réagir.

7. Procédé suivant les revendications 1 à 6, caractérisé en ce qu'on conduit la réaction à des températures comprises dans l'intervalle de 20 à 160° C.